# EUROPEAN PATENT APPLICATION

(11) **EP 1 243 647 A1**
(43) Date of publication of application: **25.09.2002**
(21) Application number: 00985799.6
(22) Date of filing: 20.12.2000
(51) Int. Cl.: C12N 15/09, C12N 1/21, C12N 9/10, C12P 19/18

(54) **MODIFIED ALPHA-1,2-FUCOSYLTRANSFERASE GENE AND PROCESS FOR PRODUCING ALPHA-1,2-FUCOSYLTRANSFERASE AND FUCOSE-CONTAINING SUGAR CHAIN**

(30) Priority: 21.12.1999 JP 36224399
(71) Applicant: KYOWA HAKKO KOGYO CO., LTD., Chiyoda-ku, Tokyo 100-8185 (JP)
(72) Inventor: ENDO, Tetsuo, c/o Kyowa Hakko Kogyo Co. Ltd., Machida-shi Tokyo 194-8533 (JP); KOIZUMI, Satoshi, c/o Kyowa Hakko Kogyo.,Ltd., Machida-shi Tokyo 194-8533 (JP); TABATA, Kazuhiko, c/o Kyowa Hakko Kogyo Co.,Ltd., Hofu-shi Yamaguchi 747-8522 (JP); OZAKI, Akio, c/o Kyowa Hakko Kogyo Co.,Ltd., Hofu-shi Yamaguchi 747-8522 (JP)
(74) Representative: VOSSIUS & PARTNER
(86) International application number: JP0009033
(87) International publication number: WO01046400

(57) **Abstract**

The present invention relates to a gene having a nucleotide sequence in which the α1,2-fucosyltransferase gene derived from Helicobacter pylori has been modified, a process for producing α1,2-fucosyltransferase using the gene, and a process for producing fucose-containing carbohydrates using a microorganism in which the enzyme has been expressed. According to the present invention, fucose-containing carbohydrates can be produced at a low cost and in large quantities by allowing a microorganism capable of producing GTP from a GTP precursor, a microorganism capable of producing GDP-fucose from a sugar and GTP, and an acceptor carbohydrate to be in contact in an aqueous medium.

## Description

### Technical Field

The present invention relates to a modified α1,2-fucosyltransferase gene, a process for producing α1,2-fucosyltransferase and a process for producing fucose-containing carbohydrate, by using the gene.

### Background Art

α1,2-fucosyltransferase genes derived from animals have so far been obtained [Proc. Natl. Acad. Sci. USA, 87, 6674 (1990), Immunogenetics, 44, 76 (1996), J. Biol. Chem., 264, 3436 (1989), J. Biol. Chem., 264, 11158 (1989), J. Biol. Chem., 267, 2737 (1992), J. Biol. Chem., 270, 8844 (1995), J. Biol. Chem., 270, 4640 (1995), J. Biochem., 118, 541 (1995), J. Biol. Chem., 271, 16975 (1996)], and however, there has been no report on expressing the gene as a protein which has α1,2-fucosyltransferase activity in a microorganism such as those of Escherichia coli.

On the other hand, in microorganisms, an α1,2-fucosyltransferase gene has been obtained from Helicobacter pylori and there has been a report on the expression of al,2-fucosyltransferase in Escherichia coli using the gene. However, the enzyme activity is very weak even the gene is put under the regulation of a strong promoter [Microbiology, 145, 3245 (1999)].

Fucose-containing sugar chains had been known as blood group antigen sugar chains. In recent years, it has been clarified that those sugar chains undergo structural changes with canceration of cells [Anal. Biochem., 251, 89 (1997)]. Thus, their application as a tumor marker and pharmaceuticals is expected. On the other hand, oligosaccharides are abundantly contained in human milk and fucose-containing sugar chains (fucosyllactose is one of the main components) occupy more than 70% of total oligosaccharides [Glycobiology, 8, 615(1998)]. The sugar chain having Fucα1-2Gal structure that is also contained in those oligosaccharides is known to inhibit the infection of Candida albicans [Infect. Immun., 59, 1650 (1991)], and therefore, fucose-containing sugar chains are considered to be a promising candidate for a safe preventive against infections.

As to the production of a fucose-containing sugar chain such as fucosyllactose, the extraction method from human milk [J. Chromatogr., 211, 170 (1981)], the production method using transgenic animals, [J. Biol. Chem., 270, 29515 (1995), USP 5,700,671] and the method using an enzyme [USP 5,583,042] have been reported, and however, any of these methods involves problems in view of the production cost or productivity and no industrial production method has been established yet.

### Disclosure of the Invention

An object of the present invention is to provide an industrial process for producing fucose-containing carbohydrates.

The present inventors have made an intensive investigation aiming at attaining the above object. As a result, they have succeeded in microbiologically producing, in large amount, α1,2-fucosyltransferase, of which mass expression as an active protein had been regarded to be difficult in a microorganism such as those of Escherichia coli, using a microorganism by modifying the nucleotide sequence of DNA encoding the enzyme and have established an industrial process for producing fucose-containing carbohydrates using the enzyme. Furthermore, through the examination of the properties of the enzyme, they have found that the enzyme uses not only Lewis X and N-acetyllactosamine as acceptor sugar chains but also lactose as an acceptor. The present invention has been completed on the basis of this finding.

The present invention relates to the following (1)-(29).
(1) A DNA encoding a protein having α1,2-fucosyltransferase activity,
   said DNA having a nucleotide sequence wherein one or more nucleotide residues in one or more members selected from the group consisting of (a) sequences, (b) sequences and (c) sequences have been deleted, substituted or added:
   (a) poly-C sequences,
   (b) TAA-analogous repeat sequences,
   (c) AAAAAAG sequences, and
   (a), (b) and (c) sequences being partial in a DNA encoding a protein having α1,2-fucosyltransferase activity derived from Helicobacter pylori.
(2) The DNA according to (1), wherein the substitution is a substitution in which one or more codons are modified into codons which are used with a high frequency in a host cell in which the DNA is expressed.
(3) A DNA encoding a protein having α1,2-fucosyltransferase activity,
   wherein said DNA is obtained by modifying one or more codons which are partial in a DNA encoding a protein having α1,2-fucosyltransferase activity derived from Helicobacter pylori into codons which are used with a high frequency in a host cell in which said DNA is expressed.
(4) The DNA according to (3), wherein the modification is a modification in one or more members selected from the group consisting of (a) sequences, (b) sequences and (c) sequences selected from:
   (a) poly-C sequences,
   (b) TAA-analogous repeat sequences,
   (c) AAAAAAG sequences, and
   (a), (b) and (c) sequences being partial in a DNA encoding a protein having α1,2-fucosyltransferase activity derived from Helicobacter pylori.
(5) The DNA according to (3), wherein the DNA comprising a nucleotide sequence which is obtained by modifying all codons into codons which are used with a high frequency in a host cell in which said DNA is expressed.
(6) A DNA comprising the nucleotide sequence represented by SEQ ID NO: 1.
(7) A DNA comprising a nucleotide sequence wherein one or more nucleotide residues have been deleted, substituted or added in the DNA represented by the nucleotide sequence shown in SEQ ID NO: 1 and encoding a protein having α1,2-fucosyltransferase activity.
(8) A recombinant DNA which is obtained by ligating DNA according to any one claims (1) to (7) with a vector.
(9) A transformant which is obtained by introducing the recombinant DNA according to claim (8) into a host cell.
(10) The transformant according to (9), wherein the host cell is a microorganism.
(11) The transformant according to (10), wherein the microorganism is a microorganism belonging to the genus Escherichia.
(12) The transformant according to (11), wherein the microorganism belonging to the genus Escherichia is Escherichia coli.
(13) A process for producing a protein having α1,2-fucosyltransferase activity which comprises culturing a transformant according to any one of (9) to (12) in a medium, allowing the protein having α1,2-fucosyltransferase activity to be formed and accumulated in the culture and recovering the protein from the culture.
(14) A process for producing a fucose-containing carbohydrate which comprises allowing a culture of a transformant according to any one of (9) to (12) or a treated matter thereof as an enzyme source, an acceptor carbohydrate, and guanosine diphosphofucose (hereinafter referred to as GDP-fucose) to be present in an aqueous medium; transferring fucose to the acceptor carbohydrate by α1,2 linkage to form and accumulate the fucose-containing carbohydrate in the aqueous medium; and recovering the fucose-containing carbohydrate from the aqueous medium.
(15) The process for producing a fucose-containing carbohydrate according to (14), which comprises allowing the following enzyme sources;
   (a) a culture of a microorganism capable of forming guanosine-5'-triphosphate (hereinafter referred to as GTP) from a precursor of GTP or a treated matter thereof,
   (b) a culture of a microorganism capable of forming GDP-fucose from a sugar and GTP or a treated matter thereof, and
   (c) a culture of a transformant selected from the transformant according to any one of (9) to (12) or a treated matter thereof,
   the precursor, a sugar, and an acceptor carbohydrate to be present in an aqueous medium;
   allowing the fucose-containing carbohydrate to be formed and accumulated in the aqueous medium; and
   recovering the fucose-containing carbohydrate from the aqueous medium.
(16) The process for producing a fucose-containing carbohydrate according to (14) or (15), wherein the acceptor carbohydrate is a carbohydrate containing an oligosaccharide consisting of 10 or less monosaccharides having galactose at its non-reducing terminal.
(17) The process for producing a fucose-containing carbohydrate according to (16), wherein the oligosaccharide is lactose, N-acetyllactosamine, Lewis X or Lewis a.
(18) The process for producing a fucose-containing carbohydrate according to (14) or (15), wherein the fucose-containing carbohydrate is fucosyllactose, fucosyl-N-acetyllactosamine, Lewis Y or Lewis b.
(19) The process for producing a fucose-containing carbohydrate according to (14) or (15), wherein the treated matter of the culture is a concentrated culture, dried culture, a cell obtained by centrifuging the culture, a cell obtained by drying or freeze drying the cell, a product obtained by treating the cell with a surfactant or by ultrasonication or mechanical friction of the cell or by treating the cell with a solvent or an enzyme or by protein-fractionation or immobilization of the cell, or an enzyme preparation obtained by extraction from the cell.
(20) The process for producing a fucose-containing carbohydrate according to (15), wherein the precursor is guanine, xanthine, hypoxanthine, guanosine, xanthosine, inosine, guanosine-5'-monophosphate, xanthosine-5'-monophosphate or inosine-5'-monophosphate.
(21) The process for producing a fucose-containing carbohydrate according to (15), wherein the sugar is a sugar selected from the group consisting of glucose, fructose and mannose.
(22) The process for producing a fucose-containing carbohydrate according to (15), wherein the microorganism capable of forming GTP from a precursor is a microorganism selected from microorganisms belonging to the genus Corynebacterium.
(23) The process for producing a fucose-containing carbohydrate according to (22), wherein the microorganisms belonging to the genus Corynebacterium are Corynebacterium ammoniagenes.
(24) The process for producing a fucose-containing carbohydrate according to (15), wherein forming GDP-fucose from a sugar and GTP is carried out by one or more microorganisms.
(25) The process for producing a fucose-containing carbohydrate according to (24), wherein the microorganisms are selected from microorganisms belonging to the genus Echerichia or Corynebacterium.
(26) The process for producing a fucose-containing carbohydrate according to (25), wherein the microorganisms belonging to the genus Escherichia are Escherichia coli.
(27) The process for producing a fucose-containing carbohydrate according to (25), wherein the microorganisms belonging to the genus Corynebacterium are Corynebacterium ammoniagenes.
(28) The process for producing a fucose-containing carbohydrate according to (15), wherein the microorganism capable of forming GDP-fucose from a sugar and GTP is a microorganism having a strong activity of one or more enzymes selected from the group consisting of glucokinase, phosphomannomutase, mannose-1-phosphoguanylyltransferase, phosphoglucomutase, phosphofructokinase, GDP-mannose 4,6-dehydratase, and GKDM epimerase/reductase.
(29) The process for producing a fucose-containing carbohydrate according to (28), wherein the microorganism comprises one or more microorganisms containing recombinant DNA which comprises a DNA fragment containing one or more genes selected from the group consisting of a gene encoding glucokinase (hereinafter referred to as glk gene), a gene encoding phosphomannomutase (hereinafter referred to as manB gene), a gene encoding mannose-1-phosphoguanyltransferase (hereinafter referred to as manC gene), a gene encoding phosphoglucomutase (hereinafter referred to as pgm gene), a gene encoding phosphofructokinase (hereinafter referred to as-pfk gene), a gene encoding GDP-mannose 4,6-dehydratase (hereinafter referred to as gmd gene), and a gene encoding GKDM epimerase/reductase (hereinafter referred to as wcaG gene) and a vector.
(30) The process for producing a fucose-containing carbohydrate according to (29), wherein the glk gene, the manB gene, the manC gene, the pgm gene, the pfk gene, the gmd gene and the wcaG gene are genes derived from Escherichia coli.

The DNA of the present invention is DNA obtained by modifying the DNA encoding a protein having α1,2-fucosyltransferase activity derived from Helicobacter pylori and includes, for example, the following DNA:
(1) DNA which encodes a protein having α1,2-fucosyltransferase activity derived from Helicobacter pylori, said DNA having a nucleotide sequence wherein one or more nucleotide residues have been deleted, substituted or added in one or more nucleotide sequences selected from the group consisting of:
   (a) a poly-C sequence which is regarded to be a hyper mutable region,
   (b) a TAA-analogous repeat sequence which is regarded to be a mutation hot-spot, and
   (c) an AAAAAAG sequence which is regarded to be susceptible to frameshift which are partial in the DNA and encoding a protein having α1,2-fucosyltransferase activity;
(2) The DNA according to the above (1), wherein the substitution is a substitution in which nucleotides are modified into a codon which is used with a high frequency in a host cell in which the DNA according to (1) is expressed;
(3) The DNA which encodes a protein having α1,2-fucosyltransferase activity derived from Helicobacter pylori, said DNA having a nucleotide sequence which is obtained by modifying one or more codons into a codon which is used with a high frequency in a host cell in which the DNA is expressed;
(4) The DNA according to the above (3) wherein the modification is a modification in one or more nucleotide sequences selected from the group consisting of:
   (a) a poly-C sequence which is regarded to be a hyper mutable region,
   (b) a TAA-analogous repeat sequence which is regarded to be a mutation hot-spot, and
   (c) an AAAAAAG sequence which is regarded to be susceptible to frameshift which are partial in the DNA encoding a protein having α1,2-fucosyltransferase activity derived from Helicobacter pylori;
(5) The DNA according to the above (3), wherein said DNA which encodes a protein having α1,2-fucosyltransferase activity derived from Helicobacter pylori and has a nucleotide sequence which is obtained by modifying all codons into codons which are used with a high frequency in a host cell in which the DNA is expressed; and
(6) DNA comprising a nucleotide sequence wherein one or more nucleotide residues have been deleted, substituted or added in the DNA which comprises the nucleotide sequence according to any one of the above (1) to (5); encoding a protein having α1,2-fucosyltransferase activity; and having a nucleotide sequence which is different from the nucleotide sequence of the α1,2-fucosyltransferase gene derived from Helicobacter pylori.

The poly-C sequence described in the above (a) refers to a nucleotide sequence containing at least 8 C residues consecutively and the TAA-analogous repeat sequence described in the-above (b) refers to a nucleotide sequence in which a sequence selected from the group consisting of TAA, GAA, AAA, TAG and TGA is repeated at least for three times consecutively in any order.

As the host cell according to the above (2) to (5), any bacterial cells, yeast cells, animal cells, insect cells, plant cells, etc. that are capable of expressing the DNA of the present invention can be used. The codon that is used with a high frequency refers to a codon which is used with a frequency of at least 10%, preferably 20% or more among codons for each amino acid in the host cell.

The above-mentioned DNA in which one or more nucleotide residues have been deleted, substituted or added can be obtained by site-directed mutagenesis described in Molecular Cloning, A Laboratory Manual, Second Edition (1989) (hereinafter referred to as Molecular Cloning, Second Edition); Current Protocols in Molecular Biology, John Wiley & Sons (1987-1997) (hereinafter referred to as Current Protocols in Molecular Biology); etc. The number of amino acid residues to be deleted, substituted or added in the protein encoded by the DNA into which the mutation is introduced is not specifically limited, but is a number that can be deleted, substituted or added in accordance with a known method including the above-mentioned site-directed mutagenesis and is preferably within the range of 1 to 20, preferably 1 to 15, more preferably 1 to 5.

In order for the DNA of the present invention to encode a protein having α1,2-fucosyltransferase activity, it is preferred that the DNA into which the mutation has been introduced has at least 60%, and generally 80% or more homology to the amino acid sequence shown in the SEQ ID No: 1, when calculated using an analyzing software such as BLAST [J. Mol. Biol., 215, 403 (1990)] and FASTA (Method in Enzymology, 183, 63-69).

The DNA of the present invention can be prepared by site-directed mutagenesis described in, for example, Molecular Cloning, Second Edition; Current Protocols in Molecular Biology; Nucl. Acids Res., 10, 6487 (1982); Proc. Natl. Acad. Sci. USA, 79, 6409 (1982); Gene, 34, 315 (1985); Nucl. Acids Res., 13, 4431 (1985); Proc. Natl. Acad. Sci. USA, 82, 488 (1985), etc., using DNA encoding a protein having α1,2-fucosyltransferase activity derived from Helicobacter pylori. The DNA can also be prepared using synthetic DNA, for example, according to the following method: (1) DNA is artificially synthesized by PCR in accordance with a conventional method [PCR Protocols, Humana Press, (1993), etc.]; and (2) around 100 bp synthetic DNAs for both sense strand and antisense strand are prepared so as to cover the full length of the target DNA and, after annealing, ligate them with a DNA ligase.

Proteins having α1,2-fucosyltransferase activity can be produced using the DNA of the present invention, for example, by preparing recombinant DNA by ligating the DNA encoding the protein which has been prepared according to the above method with vector DNA in accordance with the method described in Molecular Cloning, Second Edition; transforming a cell according to the method described in Molecular Cloning, Second Edition by using the recombinant DNA; culturing the transformant cell in a suitable medium in which the transformant cell is capable of growing; allowing the protein to form and accumulate in the culture; and recovering the protein from the culture. The protein can be recovered from the culture by solubilizing the protein, followed by isolation and purification by ion exchange, gel filtration or hydrophobic chromatography or a combination of these chromatographic methods.

The fucose-containing carbohydrate of the present invention can be obtained by using the culture of a transformant that produces the protein or a treated matter of the culture or the purified protein as an enzyme source and allowing the enzyme source, an acceptor carbohydrate and GDP-fucose to be present simultaneously in an aqueous medium.

The fucose-containing carbohydrate can also be obtained by using GDP-fucose that can be prepared by allowing a culture of a microorganism capable of producing GTP from a precursor of GTP or a treated matter of the culture and a culture consisting of cultures of one or more microorganisms capable of producing GDP-fucose from a sugar and GTP or a treated matter of the culture to be present simultaneously in an aqueous medium. The precursor referred to hereinabove is not limited so long as it is converted to GTP by the microorganism. Preferred examples include guanine, xanthine, hypoxanthine, guanosine, xanthosine, inosine, guanosine-5'-monophosphate, xanthosine-5'-monophosphate and inosine-5'-monophosphate. The sugar is also not limited so long as it can be converted to GDP-fucose and is preferably selected from the group consisting of glucose, fructose and mannose. Furthermore, any acceptor carbohydrate that can be the substrate for the protein having α1,2-fucosyltransferase activity encoded by the DNA of the present invention can be used. Preferred examples include sugars having galactose at the non-reducing terminal, and more preferably, lactose, N-acetyllactosamine, Lewis X and Lewis a.

As the microorganism capable of producing GTP from its precursor, any microorganisms that have such ability can be used without limitation. Preferably, microorganisms belonging to the genus Corynebacterium, and more preferably, Corynebacterium ammoniagenes are mentioned as examples. Furthermore, examples of the microorganism capable of producing GDP-fucose from a sugar and GTP include microorganisms having a strong activity of one or more enzymes selected from the group consisting of glucokinase, phosphomannomutase, mannose-1-phosphoguanyltransferase, phosphoglucomutase, phosphofructokinase, GDP-mannose 4,6-dehydratase, and GKDM epimerase/reductase, preferably, microorganisms consisting of one or more microorganisms containing recombinant DNA which comprises a DNA fragment containing a gene of one or more enzymes selected from the group consisting of glucokinase, phosphomannomutase, mannose-1-phosphoguanyltransferase, phosphoglucomutase, phosphofructokinase, GDP-mannose 4,6-dehydratase, and GKDM epimerase/reductase and a vector and more preferably, one or more recombinant Escherichia coli containing a glk gene, manB gene, manC gene, pgm gene, pfk gene, gmd gene, and wcaG gene derived from Escherichia coli.

The fucose-containing carbohydrate formed can be recovered by a conventional chromatography using active carbon, ion exchange resin, etc.

The present invention is described in detail below.

### [1] Preparation of a transformant expressing a protein having α1,2-fucosyltransferase activity

### (1)Preparation of the DNA of the present invention

The DNA of the present invention can be prepared according to the following method. First, an amino acid sequence of α1,2-fucosyltransferase is selected. Any amino acid sequences having the enzyme activity, for example, the amino acid sequence of α1,2-fucosyltransferase derived from Helicobacter pylori, which is shown in SEQ ID NO: 2 and registered with the database of GenBank, etc. can be used. Next, a DNA encoding a protein having the enzyme activity is designed using codons that are used with a high frequency in a host cell in which the enzyme is to be expressed. For example, when Escherichia coli is used as a host cell, the DNA of the invention can be designed by converting the selected amino acid sequence of the enzyme to a DNA sequence so as to contain the codon most frequently used, taking into consideration the frequency with which codons are used in the nucleotide sequence of the Escherichia coli gene [Codon Usage database at kazusa (http://www.kazusa.or.jp/codon/)].

When the selected amino acid sequence of α1,2-fucosyltransferase is derived from Helicobacter pylori, it is also possible to design DNA which can be obtained by deleting, substituting or adding one or more nucleotide residues in nucleotide sequences selected from the group consisting of (i) poly-C sequence, (ii) TAA-analogous repeat sequence, and (iii) AAAAAAG sequence, which are partial in the DNA encoding the amino acid sequence, and encodes a protein having the enzyme activity. For example, when the above-mentioned Helicobacter pylori is Helicobacter pylori UA802, the nucleotide sequences corresponding to the above (i), (ii), and (iii) are, for example, those shown by the nucleotide numbers 397-408, 411-434, and 430-436 and 552-558 in SEQ ID No: 27, respectively.

The above substitution can also be carried out by converting codons in the nucleotide sequence selected from the group consisting of the above (i), (ii), and (iii) to the most frequently used codons in a host cell in which the DNA of the invention is to be expressed, for example, a substitution of the nucleotide sequences shown by the nucleotide numbers 397-408, and 411-434 in the nucleotide sequence defined by SEQ ID NO: 1 for such codons is mentiond.

An example of the DNA designed in the above manner is DNA having the nucleotide sequence shown in SEQ ID NO: 1, which is designed on the basis of the α1,2-fucosyltransferase gene derived from Helicobacter pylori UA802.

Next, synthetic DNAs comprising a 40- to 150-base length from the 5'-terminal side are synthesized on the basis of the designed nucleotide sequence using an automated DNA synthesizer (e.g., Model 8905 DNA synthesizer, PerSeptive Biosystems) so that adjacent sense strand and antisense strand synthetic DNAs may become overlapping by 10 to 100 bases and that a sense-strand and an antisense strand may be alternated. Examples of such synthetic DNAs are those having the nucleotide sequences shown in SEQ ID NOS: 3 to 16, which are designed on the basis of the DNA comprising the nucleotide sequence shown in SEQ ID NO: 1. Using the thus synthesized DNAs, the DNA of the present invention is artificially synthesized by PCR in accordance with a conventional method [e.g. the method described in PCR Protocols, Humana Press (1993)]. PCR may be carried out under any conditions so long as PCR using the synthetic DNAs provides an amplified fragment having the same length as that of the DNA of the present invention, which forms for design of the synthetic DNA. For example, when DNAs having the nucleotide sequences shown in SEQ ID NOS: 3 to 16 are used, PCR is carried out by 30 cycles, one cycle consisting of reaction at 94°C for 30 seconds, reaction at 50°C for 30 seconds and reaction at 74°C for 60 seconds.

It is possible to easily clone the DNA of the present invention in a vector by introducing a recognition sequence of an appropriate restriction enzyme to 5'-terminals of the synthetic DNAs located at both ends. Examples of such synthetic DNAs are a set of DNAs having the nucleotide sequences shown in SEQ ID NOS: 17 and 18, respectively, which can be used in carrying out the above PCR using DNAs having the nucleotide sequences shown in SEQ ID NOS: 3 to 16.

### (2) Cloning of the DNA of the present invention and confirmation of its nucleotide sequence

The DNA of the invention prepared in the above (1) is ligated with a vector as it is or after cleavage with suitable restriction enzymes.

As a vector with which the DNA is ligated, either phage vectors or plasmid vectors can be used so long as they are capable of autonomous replication in Eschrichia coli K12. Examples of suitable vectors are ZAP Express [Stratagene, Strategies, 5, 58 (1992)], pBluescript II SK(+) [Nucleic Acids Research, 17, 9494 (1989)], λ zap II (Stratagene), λgt10 and λgt11 [DNA Cloning, A Practical Approach, 1, 49 (1985)], λ TriplEx (Clontech Laboratories, Inc.), λ BlueMid (Clontech Laboratories, Inc.), λExCell (Pharmacia), pT7T318U (Pharmacia), pcD2 [Mol. Cell. Biol., 3, 280 (1983)], and pUC18 [Gene, 33, 103 (1985)].

Any microorganisms belonging to Escherichia coli can be used to provide a host cell into which the recombinant DNA obtained by ligating the DNA of the invention prepared in the above (1) to the vector is introduced. Examples of suitable host cells include cells of Escherichia coli XL1-Blue MRF' [Stratagene, Strategies, 5, 81 (1992)], Escherichia coli C600 [Genetics, 39, 440 (1954)], Escherichia coli Y1088 [Science, 222, 778 (1983)], Escherichia coli Y1090 [Science, 222, 778 (1983)], Escherichia coli NM522 [J. Mol. Biol., 166, 1 (1983)], Escherichia coli K802 [J. Mol. Biol., 16, 118 (1966)], and Escherichia coli JM105 {Gene, 38, 275 (1985)}.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 2483942/88) and electroporation [Nucleic Acids Research, 16, 6127 (1988)].

After extracting the recombinant DNA from the transformant obtained in the above manner, the nucleotide sequence of the DNA of the present invention contained in the recombinant DNA can be determined. Determination of the nucleotide sequence can be carried out according to the method usually used for determining nucleotide sequences, for example, the dideoxy method [Proc. Natl. Acad. Sci. USA, 74, 5463 (1977)] or using a nucleotide sequencer, for example, 373A DNA sequencer (Perkin-Elmer). Thus, the DNA of the present invention prepared in the above (1) can be identified with the DNA that has been designed to provide the basis of the DNA synthesis.

An example of the transformant strains containing the recombinant DNA obtained in the above manner is Escherichia coli NM522/pGT35 which contains plasmid DNA having the nucleotide sequence shown in SEQ ID NO: 1.

### [2] Preparation of the protein having α1,2-fucosyltransferase activity using the DNA of the present invention

The protein having α1,2-fucosyltransferase activity encoded by the DNA of the present invention can be produced by expressing the DNA in a host cell using the method described in Molecular Cloning, Second Edition or Current Protocols in Molecular Biology according to, for example, the following method.

On the basis of the DNA of the present invention, a DNA fragment of an appropriate length comprising a region encoding the protein is prepared as required and the DNA fragment is inserted downstream to a promoter region in an appropriate expression vector to construct recombinant DNA. The protein having α1,2-fucosyltransferase activity can be produced by introducing the recombinant DNA into a host cell suited for the expression vector to prepare a transformant, culturing the transformant in a medium, and allowing the protein to form and accumulate in the culture.

As the host cell, any bacterial cells, yeast cells, animal cells, insect cells, plant cells, etc. that are capable of expressing the target gene can be used.

Expression vectors that can be employed are those capable of autonomous replication or integration into chromosomal DNA in the above host cells and containing a promoter at a region where the DNA encoding the protein of the present invention can be transcribed.

When a procaryotic cell such as a bacterial cell is used as the host cell, it is preferred that recombinant DNA comprising DNA encoding the protein of the present invention is capable of autonomous replication in the procaryotic cell and, at the same time, is a vector which comprises a promoter, a ribosome binding sequence, the DNA of the present invention, and a transcription termination sequence. The vector may further comprise a gene regulating the promoter.

Examples of suitable expression vectors include pBTrp2, pBTac1 and pBTac2 (all available from Boehringer Mannheim), pKK233-2 (Pharmacia), pSE280 (Invitrogen), pGEMEX-1 (Promega), pQE-8 (QIAGEN), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (Stratagene), pTrs30 [prepared from Escherichia coli JM109/pTrs30 (FERM BP-5407)], pTrS32 [prepared from Escherichia coli JM109/pTrS32 (FERM BP-5408)], pGHA2 [prepared from Escherichia coli IGHA2 (FERM P-400), Japanese Published Unexamined Patent Application No. 221091/85], pGKA2 [prepared from Escherichia coli IGKA2 (FERM BP-6798) Japanese Published Unexamined Patent Application No. 221091/85], pTerm2 (US4686191, US4939094, US5160735), pSupex, pUB110, pTP5, pC194, pEG400 [J. Bacteriol., 172, 2392 (1990)], pGEX (Pharmacia), and pET system (Novagen).

As the promoter, any promoters capable of functioning in host cells can be used. For example, promoters derived from Escherichia coli or phage, such as trp promoter (Pₜᵣₚ), lac promoter, P_{L} promoter, P_{R} promoter and T7 promoter can be used. Artificially modified promoters such as a promoter in which two Pₜᵣₚs are combined in tandem (Pₜᵣₚ x 2), tac promoter, lacT7 promoter and letI promoter, etc. can also be used.

It is preferred to use plasmid in which the distance between the Shine-Dalgarno sequence and the initiation codon is adjusted to an appropriate length (e.g.-, 6-18 bases).

In the case of the recombinant DNA of the present invention, the transcription termination sequence is not essential for the expression of the DNA of the invention, but it is preferred that the transcription termination sequence lie immediate downstream the structural gene.

Examples of suitable host cells are cells of microorganisms belonging to the genera Escherichia, Serratia, Bacillus, Brevibacterium, Corynebacterium, Microbacterium, Pseudomonas, etc., specifically, those of Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli GI698, Escherichia coli TB1, Serratia ficaris, Serratia fonticola, Serratia liguefaciens, Serratia marcescens, Bacillus subtilis, Bacillus amyloliquefacines, Corynebacterium ammmoniagenes, Brevibacterium immariophilum ATCC14068, Brevibacterium saccharolyticum ATCC14066, Brevibacterium flavum ATCC14067, Brevibacterium lactofermentum ATCC13869, Corynebacterium glutamicum ATCC13032, Corynebacterium glutamicum ATCC13869, Corynebacterium acetoacidophilum ATCC13870, Microbacterium ammoniaphilum ATCC15354, Pseudomonas putida, and Pseudomonas sp. D-0110.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and the methods described in Gene, 17, 107 (1982) and Mol. Gen. Genet., 168, 111 (1979).

When a yeast cell is used as the host cell, YEP13 (ATCC37115), YEp24 (ATCC37051), YCp50 (ATCC37419), pHS19, pHS15 etc. can be used as the expression vector.

As the promoter, any promoters capable of functioning in yeast cells can be used. Suitable promoters include promoters of hexosekinase and other glycolytic genes, PHO5 promoter, PGK promoter, GAP promoter, ADH promoter, gal 1 promoter, gal 10 promoter, heat shock polypeptide promoter, MFα1 promoter, CUP1 promoter, etc.

Examples of suitable host cells include cells of microorganism strains belonging to the genus Saccharomyces, Schizosaccharomyces, Kluyveromyces, Trichosporon, Schwanniomyces, Pichia or Candida, specifically, the species Saccharomyces cerevisiae, Schizosaccharomyces pombe, Kluyveromyces lactis, Trichosporon pullulans, Schwanniomyces alluvius, or Candida utilis.

Introduction of the recombinant DNA can be carried out by any of the methods for introducing DNA into yeast cells, for example, electroporation [Methods Enzymol., 194, 182 (1990)], the spheroplast method [Proc. Natl. Acad. Sci. USA, 75, 1929 (1978)], the lithium acetate method [J. Bacteriology, 153, 163 (1983)] and the method described in Proc. Natl. Acad. Sci. USA, 75, 1929 (1978).

When an animal cell is used as the host cell, pcDNAI, and pCDM8 (both available from Funakoshi), pAGE107 [Japanese Published Unexamined Patent Application No. 22979/91; Cytotechnology, 3, 133 (1990)], pAS3-3 (Japanese Published Unexamined Patent Application No. 227075/90) pCDM8 [Nature, 329, 840 (1987)], pcDNAI/Amp (Invitrogen), pREP4 (Invitrogen), pAGE103 [J. Biochem., 101, 1307 (1987)], pAGE210, etc. can be used as the expression vector.

As the promoter, any promoters capable of functioning in animal cells can be used. Suitable promoters include the promoter of IE (immediate early) gene of cytomegalovirus (CMV), SV40 early promoter, the promoter of a retrovirus, metallothionein promoter, heat shock promoter, SRα promoter, etc. The enhancer of IE gene of human CMV may be used in combination with the promoter.

Examples of suitable host cells are human-derived Namalwa cells, monkey-derived COS cells, Chinese hamster-derived CHO cells, and HBT5637 (Japanese Published Unexamined Patent Application No. 299/88).

Introduction of the recombinant DNA into animal cells can be carried out by any of the methods for introducing DNA into animal cells, for example, electroporation [Cytotechnology, 3, 133 (1990)], the calcium phosphate method (Japanese Published Unexamined Patent Application No. 227075/90), lipofection [Proc. Natl. Acad. Sci. USA, 84, 7413 (1987)], and the method described in Virology, 52, 456 (1973).

When an insect cell is used as the host cell, the polypeptide can be expressed by using the methods described in Current Protocols in Molecular Biology; Baculovirus Expression Vectors, A Laboratory Manual, W. H. Freeman and Company, New York"(1992); Bio/Technology, 6, 47 (1988), etc.

That is, the recombinant gene transfer vector and a baculovirus are cotransfected into an insect cell to obtain a recombinant virus in the culture supernatant of the insect cell and then an insect cell is infected with the recombinant virus, whereby the protein can be expressed.

Examples of the gene transfer vectors suitable for use in this method are pVL1392, pVL1393 and pBlueBacIII (products of Invitrogen).

An example of the baculovirus is Autographa californica nuclear polyhedrosis virus which is a virus infecting insects belonging to the family Barathra.

Examples of the insect cells are Sf9 and Sf21 which are ovarian cells of Spodoptera frugiperda [Baculovirus Expression Vectors, A Laboratory Manual, W. H. Freeman and Company, New York (1992)], and High 5 which is an ovarian cell of Trichoplusia ni (Invitrogen).

Cotransfection of the above recombinant gene transfer vector and the above baculovirus into an insect cell for the preparation of the recombinant virus can be carried out by the calcium phosphate method (Japanese Published Unexamined Patent Application No. 227075/90), lipofection [Proc. Natl. Acad. Sci. USA, 84, 7413 (1987)], etc.

When a plant cell is used as the host cell, Ti plasmid, tobacco mosaic virus vector, etc. are useful expression vectors.

As the promoter, any promoters capable of functioning in plant cells can be used. Suitable promoters include 35S promoter of cauliflower mosaic virus (CaMV), rice actin 1 promoter, etc.

Examples of suitable host cells are cells of plants such as tobacco, potato, tomato, carrot, soybean, rape, alfalfa, rice, wheat, and barley.

Introduction of the recombinant DNA can be carried out by any of the methods for introducing DNA into plant cells, for example, the Agrobacterium method (Japanese Published Unexamined Patent Applications Nos. 140885/84 and 70080/85, WO94/00977), electroporation (Japanese Published Unexamined Patent Application No. 251887/85) and the method using particle gun (gene gun) (Japanese Patents Nos. 2606856 and 2517813).

The protein of the invention can be produced by culturing the transformant obtained according to the above procedure in a medium, allowing the protein of the present invention to form and accumulate in the culture, and recovering the protein from the culture.

Culturing of the transformant of the invention in a medium can be carried out by conventional methods for culturing a host cell of a transformant.

When the transformant of the present invention is prepared by using a procaryote such as Escherichia coli or a eucaryote such as yeast as the host, any of natural media and synthetic media can be used for culturing the transformant insofar as it is a medium suitable-for efficient culturing of the transformant, which contains carbon sources, nitrogen sources, inorganic salts, etc. which can be assimilated by the transformant used.

As the carbon sources, any carbon sources that can be assimilated by the transformant can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of various organic or inorganic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is carried out under aerobic conditions, for example, by shaking culture or submerged spinner culture under aeration, at 15 to 40°C usually for 16 hours to 7 days. The pH is maintained preferably at 3.0 to 9.0 during the culturing. The pH adjustment is carried out by using an organic or inorganic acid, an alkali solution, urea, calcium carbonate, ammonia, etc.

If necessary, antibiotics such as ampicillin, tetracycline, and chloramphenicol may be added to the medium during the culturing.

When a microorganism transformed with a recombinant DNA comprising an inducible promoter is cultured, an inducer may be added to the medium, if necessary. For example, in the case of a microorganism transformed with recombinant DNA comprising lac promoter, isopropyl-β-D-thiogalactopyranoside or the like may be added to the medium; and in the case of a microorganism transformed with recombinant DNA comprising trp promoter, indoleacrylic acid or the like may be added.

For the culturing of the transformant prepared by using an animal cell as the host cell, generally employed media such as RPMI1640 medium [J. Am. Med. Assoc., 199, 519 (1967)], Eagle's MEM [Science, 122, 501 (1952)], Dulbecco's modified MEM [Virology, 8, 396 (1959)] and 199 medium [Proc. Soc. Biol. Med., 73, 1 (1950)], media prepared by adding fetal calf serum or the like to these media, etc. can be used as the medium.

Culturing is usually carried out at pH 6 to 8 at 30 to 40°C for 1 to 7 days in the presence of 5% CO₂.

If necessary, antibiotics such as kanamycin and penicillin may be added to the medium during the culturing.

For the culturing of the transformant prepared by using an insect cell as the host cell, generally employed media such as TNM-FH medium (Pharmingen), Sf-900 II SFM medium (Life Technologies), ExCell 400 and ExCell 405 (both JRH Biosciences) and Grace's Insect Medium [Nature, 195, 788 (1962)] can be used as the medium.

Culturing is usually carried out at pH 6 to 7 at 25 to 30°C for 1 to 5 days.

If necessary, antibiotics such as gentamicin may be added to the medium during the culturing.

The transformant prepared by using a plant cell as the host cell may be cultured in the form of cells as such or after differentiation into plant cells or plant organs. For the culturing of such transformant, generally used media such as Murashige-Skoog (MS) medium and White medium, media prepared by adding phytohormones such as auxin and cytokinin to these media, etc. can be used as the medium.

Culturing is usually carried out at pH 5 to 9 at 20 to 40°C for 3 to 60 days.

If necessary, antibiotics such as kanamycin and hygromycin may be added to the medium during the culturing.

As described above, the protein of the present invention can be produced by culturing a transformant prepared by introducing recombinant DNA comprising DNA encoding the polypeptide of the present invention into a microorganism, an animal cell or a plant cell according to a conventional culturing method, allowing the protein to form and accumulate, and recovering the protein from the culture.

The gene can be expressed either directly or as a secretory production or fusion protein expression according to the methods described in Molecular Cloning, Second Edition, etc.

The protein of the present invention may be produced intracellularly, secreted extracellularly or produced on outer membranes by host cells. Such production methods can be selected according to the kind of the host cell used or the structure of the protein to be produced.

When the protein of the present invention is produced in host cells or on outer membranes of host cells, it is possible to force the polypeptide to be secreted outside the host cells by applying the method of Paulson, et al. [J. Biol. Chem., 264, 17619 (1989)], the method of Lowe, et al. [Proc. Natl. Acad. Sci. USA, 86, 8227 (1989); Genes Develop., 4, 1288 (1990)], or the methods described in Japanese Published Unexamined Patent Application Nos. 336963/93 and 823021/94, etc.

That is, extracellular secretion of the protein of the present invention can be caused by expressing it in the form of a protein in which a signal peptide is added upstream to a polypeptide containing the active site of the protein of the present invention by the use of recombinant DNA techniques.

It is also possible to increase the protein production by utilizing a gene amplification system using a dihydrofolate reductase gene or the like according to the method described in Japanese Published Unexamined Patent Application No. 227075/90.

Furthermore, it is possible to cause animal or plant cells carrying the introduced gene to redifferentiate to construct an animal (non-human transgenic animal) or a plant (transgenic plant) having the introduced gene and produce the protein of the present invention by using these individual.

When the transformant is an animal or plant, the protein can be produced by raising or culturing the animal or plant in a usual manner, allowing the polypeptide to form and accumulate therein, and recovering the protein from the animal or plant.

The protein of the present invention can be produced by using an animal, for example, by introducing the gene into the animal in accordance with known methods [Am. J. Clin. Nutr., 63, 639S (1996); Am. J. Clin. Nutr., 63, 627S (1996); Bio/Technology, 9, 830 (1991)].

In the case of using an animal, the protein of the present invention can be produced, for example, by raising a non-human transgenic animal in which the DNA encoding the protein of the present invention has been introduced, allowing the protein to form and accumulate in the animal, and recovering the protein from the animal. The places where the protein is formed and accumulated include milk (Japanese Published Unexamined Patent Application No. 309192/88), egg, etc. of the animal. As the promoter, any promoters capable of functioning in an animal can be used. Preferred promoters include mammary gland cell-specific promoters such as α casein promoter, β casein promoter, β lactoglobulin promoter and whey acidic protein promoter.

On the other hand, to produce the protein of the invention by using a plant, for example, a process comprising culturing a transgenic plant in which the DNA encoding the protein of the invention has been introduced according to known culturing methods [Soshiki Baiyo (Tissue Culture), 20 (1994); Soshiki Baiyo (Tissue Culture), 21 (1995); Trends Biotechnol., 15, 45 (1997)], allowing the protein to form and accumulate in the plant, and recovering the protein from the plant may be used.

The protein produced by the transformant of the present invention can be isolated and purified by conventional methods for isolating and purifying enzymes. For example, when the protein of the present invention is expressed in a soluble form in cells, isolation and purification can be carried out in the following manner: after the completion of culturing, the cells are recovered by centrifugation and suspended in an aqueous buffer, followed by disruption using an ultrasonic disrupter, a French press, Manton Gaulin homogenizer, Dyno Mill, etc. to obtain a cell-free extract, which is then centrifuged. From the resulting supernatant, a purified protein preparation can be obtained by an ordinary means to isolate and purify enzymes, for example, extraction with a solvent, salting-out with ammonium sulfate, etc., desalting, precipitation with an organic solvent, anion exchange chromatography using resins such as diethylaminoethyl (DEAE)-Sepharose and DIAION HPA-75 (Mitsubishi Kasei Corporation), cation exchange chromatography using resins such as S-Sepharose FF (Pharmacia), hydrophobic chromatography using resins such. as butyl Sepharose and phenyl Sepharose, gel filtration using a molecular sieve, affinity chromatography, chromatofocusing, and electrophoresis such as isoelectric focusing, alone or in combination.

When the protein is expressed as an inclusion body in cells, the cells are similarly recovered, and disrupted, followed by centrifugation to obtain the inclusion body of the protein as a precipitate fraction, which is then solubilized with a protein-denaturing agent. The solubilized solution is diluted or dialyzed to reduce the concentration of the protein-denaturing agent, whereby the normal three-dimensional structure of the protein is restored. After carrying out these operations, a purified protein preparation can be obtained through the same isolation and purification procedures as mentioned above.

When the protein of the present invention or its derivatives such as a protein containing a sugar chain added to the protein is in the same manner as mentionded, secreted, the protein or the derivatives can be recovered from the culture supernatant. That is, the culture is treated using the above means such as centrifugation to obtain a supernatant, from which a purified preparation of the protein can be obtained through the same isolation and purification procedures as described above.

An example of the protein obtained in this manner is the protein having the amino acid sequence shown in SEQ ID NO: 2.

The protein of the present invention can also be produced by chemical synthetic methods such as the Fmoc method (the fluorenylmethyloxycarbonyl method) and the tBoc method (the t-butyloxycarbonyl method). Furthermore, the protein can be chemically synthesized by using peptide synthesizers available from Advanced ChemTech, Perkin-Elmer, Pharmacia, Protein Technology Instrument, Synthecell-Vega, PerSeptive, Shimadzu Corporation, etc.

### [3] Production of a fucose-containing carbohydrate

A fucose-containing carbohydrate can be produced in an aqueous medium using the culture of the transformant obtained by the culturing described in [2] above or treated matters thereof obtained by treating the culture by various means-as an enzyme source.

The treated matters of the culture include concentrated culture, dried culture, cells obtained by centrifuging the culture, cells obtained by drying and freeze-drying the cells, products obtained by treating the cells by various means such as treatment with a surfactant, ultrasonication, mechanical friction, treatment with a solvent, enzymatic treatment, protein fractionation and immobilization, and an enzyme preparation obtained by extracting the cells.

The enzyme source to be used for the preparation of the fucose-containing carbohydrate is used at a concentration of 0.1 mU/l to 10,000 U/l, preferably 1 mU/l to 1,000 U/l, one unit (U) being defined as the activity which forms 1 µmole of the fucose-containing carbohydrate at 37°C in one minutes.

Aqueous media useful in the preparation of the fucose-containing carbohydrate include water, buffers such as phosphate buffer, carbonate buffer, acetate buffer, borate buffer, citrate buffer and Tris buffer, alcohols such as methanol and ethanol, esters such as ethyl acetate, ketones such as acetone, amides such acetamide, etc. Also useful aqueous medium is the culture of the microorganism used as an enzyme source.

If necessary, a surfactant or an organic solvent may be added during the formation of the fucose-containing carbohydrate. Any surfactant that promotes the formation of the fucose-containing carbohydrate can be used. Suitable surfactants include nonionic surfactants such as polyoxyethylene octadecylamine (e.g., Nymeen S-215, NOF Corporation), cationic surfactants such as cetyltrimethylammonium bromide and alkyldimethyl benzylammonium chloride (e.g., Cation F2-40E, NOF Corporation), anionic surfactants such as lauroyl sarcosinate, and tertiary amines such as alkyldimethylamine (e.g., Tertiary Amine FB, NOF Corporation), which may be used alone or in combination. The surfactant is usually used at a concentration of 0.1 to 50 g/l. As the organic solvent, xylene, toluene, aliphatic alcohols, acetone, ethyl acetone, etc. may be used usually at a concentration of 0.1 to 50 ml/l.

The sugar nucleotide substrate to be use in the preparation of the fucose-containing carbohydrate, guanosinediphosphofucose (GDP-Fuc), may be a commercially available one, a reaction mixture prepared by utilizing the activity of a microorganism or the like, or a purified product obtained from such reaction mixture.

The sugar nucleotide substrate is used at a concentration of 0.1 to 500 m mol/l.

Any acceptor carbohydrate may be used as the acceptor carbohydrate for the formation of the fucose-containing carbohydrate so long as it can be a substrate for glycosyltransferase. Suitable acceptor carbohydrates include lactose, N-acetyllactosamine, Lewis X, Lewis a, Galβ1-4[Fucα1,3]GlcNAcβ1-3Galβ1-4Glc (LNFPIII), oligosaccharides consisting of 10 or less monosaccharides and having galactose or N-acetyllactosamine structure at the non-reducing terminal, etc.

The acceptor carbohydrate is used at a concentration of 0.1 to 500 m mol/l.

If necessary, an inorganic salt such as MnCl₂, β-mercaptoethanol, etc. may be added in the reaction for forming the fucose-containing carbohydrate.

The reaction for forming the fucose-containing carbohydrate is carried out in an aqueous medium at pH5 to 10, preferably pH6 to 8, at 20 to 50°C for 1 to 96 hours.

Quantitative determination of the fucose-containing carbohydrate formed in the aqueous medium can be carried out according to a known method [Kagaku to Kogyo (Chemistry and Industry), 43, 953 (1990)].

The fucose-containing carbohydrate can be recovered from the reaction mixture by an ordinary means using active carbon, ion-exchange resins, etc., and 2'-fucosyllactose, for instance, can be recovered in accordance with the method described in J. Org. Chem., 47, 5416 (1982).

Examples of the fucose-containing carbohydrates thus obtained include fucosyllactose, fucosylN-acetyllatosamine, Lewis Y, and Lewis b.

### Brief Description of the Drawings

Fig. 1 shows the structure of plasmid pGT35 expressing α1,2-fucosyltransferase gene. In the figure, Amp^{r} indicates ampicillin resistance gene; Pₜᵣₚ, trp promoter; and FTS, α1,2-fucosyltransferase gene.
Fig. 2 shows the steps for constructing plasmid pNK11 expressing glk, manB and manC. In the figure, Amp^{r} indicates ampicillin resistance gene; P_{L}, P_{L} promoter; cI857, cI857 repressor; glk, glucokinase gene; manB, phosphomannomutase gene; and manC, mannose-1-phosphoguanylyltransferase gene.
Fig. 3 shows the steps for constructing plasmid pGE19 expressing gmd. In the figure; Amp^{r} indicates ampicillin resistance gene; Pₜᵣₚ, trp promoter; P_{lac}, lac promoter; and gmd, GDP-mannose 4,6-dehydratase gene.
Fig. 4 shows the steps for constructing plasmid pGE8 expressing wcaG. In the figure, Amp^{r} indicates ampicillin resistance gene; P_{L}, P_{L} promoter; cI857, cI857 repressor; and wcaG, GKDM epimerase/reductase gene.

Certain embodiments of the present invention are illustrated in the following examples. These examples are not to be construed as limiting the scope of the invention.

### Best Modes for Carrying out the Invention

### Example 1

### Construction of a strain expressing α1,2-fucosyltransferase

The amino acid sequence of α1,2-fucosyltransferase of Helicobacter pylori (UA802) represented by the amino acid sequence shown in SEQ ID NO: 2 (GenBank: AF076779) was selected as an amino acid sequence of α1,2-fucosyltransferase and, taking into consideration the frequency with which codons are used in Escherichia coli [Codon Usage database at kazusa (http://www.Kazusa.or.jp/codon/)], converted to a DNA sequence comprising codons that are most frequently used. Thus, the DNA sequence shown in SEQ ID NO: 1 was designed. On the basis of the designed nucleotide sequence, DNAs shown by SEQ ID NOS: 3 to 16 were synthesized using Model 8905 DNA synthesizer (PerSeptive Biosystems) so that adjacent sense strand and antisense strand synthetic DNAs might have overrappinnng mutually by 20 bases and that a sense strand and an antisense strand might be alternated. Also the DNAs shown in SEQ ID NOS: 17 and 18 that contain a restriction enzyme recognition sequence for cloning to a vector at the terminals were similarly synthesized.

Each DNA was added to 50 µl of a buffer consisting of 16 m mol/l ammonium sulfate, 10 m mol/l potassium chloride, 1 m mol/l magnesium chloride, 20 m mol/l Tris-hydrochloric acid (pH 8.0), 0.1% Triton X-100, 0.001% BSA, 200 µmol/l dNTPs, and 2.5 U of KOD DNA polymerase (Toyobo) so as to have a final concentration of 0.02 µmol/l, covered with 50 µl of a mineral oil, set in a DNA thermal cycler (PJ480, PERKIN ELMER) and subjected to 30 PCR cycles, one cycle consisting of a reaction at 94°C for 30 seconds, a reaction at 50°C for 30 seconds, and a reaction at 74°C for 60 seconds, to obtain a PCR product of about 0.9 kb.

The PCR product (0.5 µg) was cleaved with restriction enzymes ClaI and HindIII and subjected to ligation reaction together with 0.2 µg of pBluescriptII SK(+)DNA (Stratagene) which had been cleaved with restriction enzymes ClaI and HindIII using a ligation kit (Takara Shuzo Co., Ltd.) at 16°C for 16 hours.

Escherichia coli NM522 was transformed using the ligation mixture according to the known method described above, spread on LB agar medium [10 g/l Bacto-tryptone (Difco Laboratories Inc.), 5 g/l yeast extract (Difco Laboratories Inc.), 5 g/l NaCl (pH 7.2)and 15 g/l agar] containing 50 µg/ml ampicillin, and cultured overnight at 30°C.

A plasmid was extracted from the colonies of transformants grown on the medium according to a conventional method, whereby plasmid pGT32 was obtained.

The plasmid pGT32 thus obtained (0.5 µg) was cleaved with restriction enzymes ClaI and SacI and about 0.9 kb ClaI-SacI DNA fragment separated by agarose gel electrophoresis, and 0.2 µg of pTrS30 which had been cleaved with restriction enzymes ClaI and SacI (Takara Shuzo Co., Ltd.) were subjected to ligation reaction using a ligation kit (Takara Shuzo Co., Ltd.) at 16°C for 16 hours.

Escherichia coli NM522 was transformed using the ligation mixture according to the known method described above, spread on LB agar medium [10 g/l Bacto-tryptone (Difco Laboratories Inc.), 5 g/l yeast extract (Difco Laboratories Inc.), 5 g/l NaCl (pH 7.2) and 15 g/l agar] containing 50 µg/ml ampicillin, and cultured overnight at 30°C.

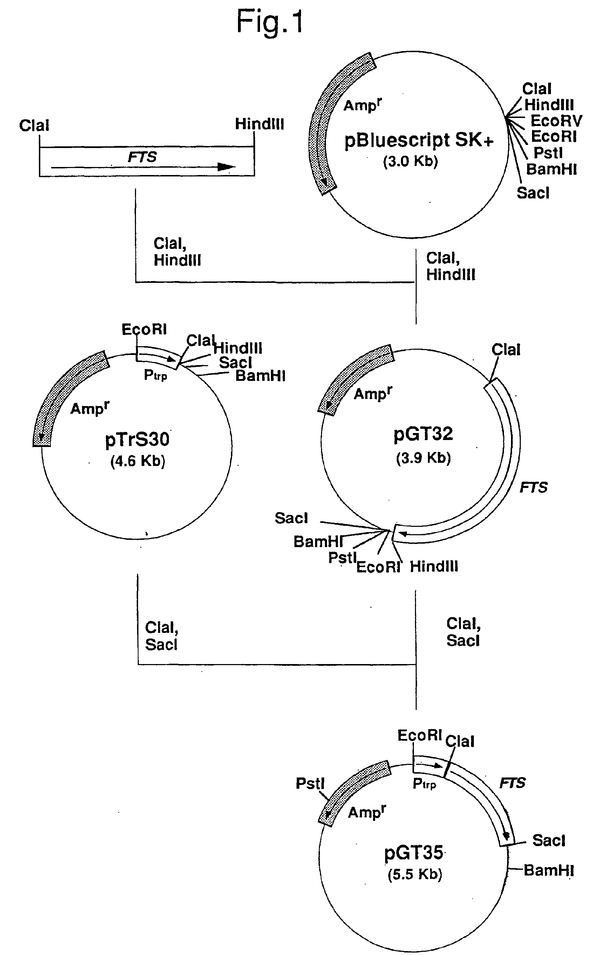

A plasmid was extracted from the colonies of transformants grown on the medium, whereby plasmid pGT35 was obtained. Upon analysis of the structure, plasmid pGT35 was revealed to have a structure in which fully synthesized 0.9 kb DNA fragment was inserted to the ClaI and SacI sites of plasmid pGT30 as shown in Fig. 1.

When the nucleotide sequence of the inserted 0.9 kb DNA fragment was determined, an open reading frame (ORF) shown in SEQ ID NO: 1 was found.

Escherichia coli NM522 was transformed with plasmid pGT35 obtained above according to a known method, spread on LB agar medium containing 50 µg/ml ampicillin, and cultured overnight at 30°C. Through the screening of the transformants grown on the medium, Escherichia coli NM522/pGT35, a strain in which α1,2-fucosyltransferase gene is expressed, was obtained.

### Example 2

### Construction of a strain in which glk, manB, manC, pgm and pfkB genes are expressed

The DNA primer shown in SEQ ID NO: 19 and the DNA shown in SEQ ID NO: 20 were synthesized by Model 8905 DNA synthesizer (PerSeptive Biosystems).

PCR was carried out using the above synthetic DNAs as a set of primers and plasmid pNT46(WO98/12343)DNA containing a glk gene as a template. That is, PCR was carried out by 30 cycles, one cycle consisting of a reaction at 94°C for one minute, a reaction at 42°C for 2 minutes and a reaction at 72°C for 3 minutes, using 40 µl of a reaction mixture comprising 1 ng of pNT46DNA, 0.5 µmol/l each of the corresponding primers, 2.5 units of Pfu DNA polymerase (Stratagene), 4 µl of a buffer for Pfu DNA polymerase (x10)(Stratagene) and 200 µmol/l each of deoxyNTPs.

One-tenth of the resulting reaction mixture was subjected to agarose gel electrophoresis to confirm that the target fragment was amplified. Thereafter, the remaining reaction mixture was mixed with an equal amount of phenol/chloroform saturated with TE [10 m mol/l Tris-HCl (pH8.0), 1 m mol/l EDTA].

After centrifuging the mixture, the obtained upper layer was mixed with a two-fold volume of cold ethanol and allowed to stand at -80°C for 30 minutes. The resulting mixture was centrifuged to obtain a DNA precipitate.

The precipitate was dissolved in 20 µl of TE and 5 µl of the solution was subjected to DNA cleavage with restriction enzymes BglII and SalI. DNA fragments were separated by agarose gel electrophoresis and 1.3 kb DNA fragment containing a glk gene was recovered using Gene Clean II Kit.

pNK7(WO98/12343) (0.2 µg), plasmid expressing manB and manC, was cleaved with restriction enzymes BamHI and SalI, DNA fragments were separated by agarose gel electrophoresis and 8.2 kb fragment was recovered in the same manner as above.

The 1.3 kb and 8.2 kb fragments were subjected to ligation reaction using a ligation kit at 16°C for 16 hours. Escherichia coli NM522 was transformed using the ligation mixture according to the known method described above, spread on LB agar medium containing 50 µg/ml ampicillin, and cultured overnight at 30°C.

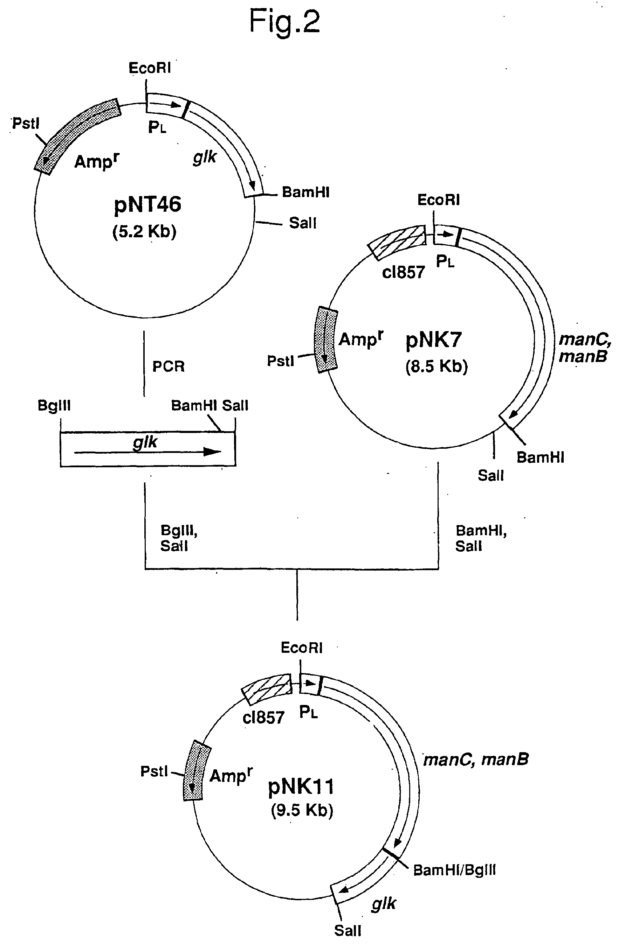

A plasmid was extracted from the colonies of transformants grown on the medium according to the known method described above, whereby plasmid pNK11, plasmid expressing glk, manB, and manC genes, was obtained. The structure of the plasmid was confirmed by digestion with restriction enzymes (Fig. 2).

Escherichia coli NM522/pNT55 (WO98/12343) was transformed using the plasmid pNK11 DNA obtained above according to a known method, spread on LB agar medium containing 50 µg/ml ampicillin and 10 µg/ml of chloramphenicol and cultured overnight at 30°C. Through the selecting of the transformants grown on the medium, Escherichia coli NM522/pNK11/pNT55, a strain in which glk, manB, manC, pgm and pfkB genes were simultaneously expressed, was obtained.

### Example 3

### Construction of a strain in which a gmd gene derived from Escherichia coli is expresssed

Escherichia coli W3110 (ATCC27325) was cultured according to the method described in Current Protocols in Molecular Biology, and then chromosomal DNA of the microorganism was isolated and purified.

PCR was carried out using DNAs shown in SEQ ID NOS: 21 and 21, respectively, which had been synthesized by using Model 8905 DNA synthesizer (PerSeptive Biosystems) as a set of primers, and 0.1 µg of chromosomal DNA of Escherichia coli W3110 (ATCC27325) as a template according to the method described in Example 1.

One-tenth of the resulting reaction mixture was subjected to agarose gel electrophoresis to confirm that the target fragment was amplified and the remaining reaction mixture was mixed with an equal amount of TE-saturated phenol/chloroform.

After centrifuging the mixture, the obtained upper layer was mixed with a two-fold volume of cold ethanol and allowed to stand at -80°C for 30 minutes. The resulting mixture was centrifuged to obtain a DNA precipitate.

The DNA precipitate was dissolved in 20 µl of TE and 5 µl of the solution was subjected to DNA cleavage with restriction enzymes HindIII and XbaI. DNA fragments were separated by agarose gel electrophoresis and 1.1 kb DNA fragment containing a glk gene was recovered using Gene Clean II Kit.

PCR was carried out using DNAs having nucleotide sequences shown in SEQ ID NOS: 23 and 24, respectively, which had been synthesized by using Model 8905 DNA synthesizer (PerSeptive Biosystems) as a set of primers, and pTrS30 (FERM BP-5407), plasmid containing trp promoter, as a template according to the method described in Example 1.

One-tenth of the resulting reaction mixture was subjected to agarose gel electrophoresis to confirm that the target fragment was amplified and the remaining reaction mixture was mixed with an equal amount of TE-saturated phenol/chloroform.

After centrifuging the mixture, the obtained upper layer was mixed with a two-fold volume of cold ethanol and allowed to stand at -80°C for 30 minutes. The resulting mixture was centrifuged to obtain a DNA precipitate.

The DNA precipitate was dissolved in 20 µl of TE and 5 µl of the solution was subjected to DNA cleavage with restriction enzymes EcoRI and XbaI. DNA fragments were separated by agarose gel electrophoresis and 0.4 kb DNA fragment was recovered in the same manner as above.

pBluescriptII SK+ (0.2 µg) was cleaved with restriction enzymes EcoRI and HindIII, DNA fragments were separated by agarose gel electrophoresis and 3.0 kb fragment was recovered in the same manner as above.

The 1.1 kb, 0.4 kb, and 3.0 kb fragments were subjected to ligation reaction using a ligation kit at 16°C for 16 hours.

Escherichia coli NM522 was transformed using the ligation mixture according to the known method described above, spread on LB agar medium containing 50 µg/l ampicillin, and cultured overnight at 30°C.

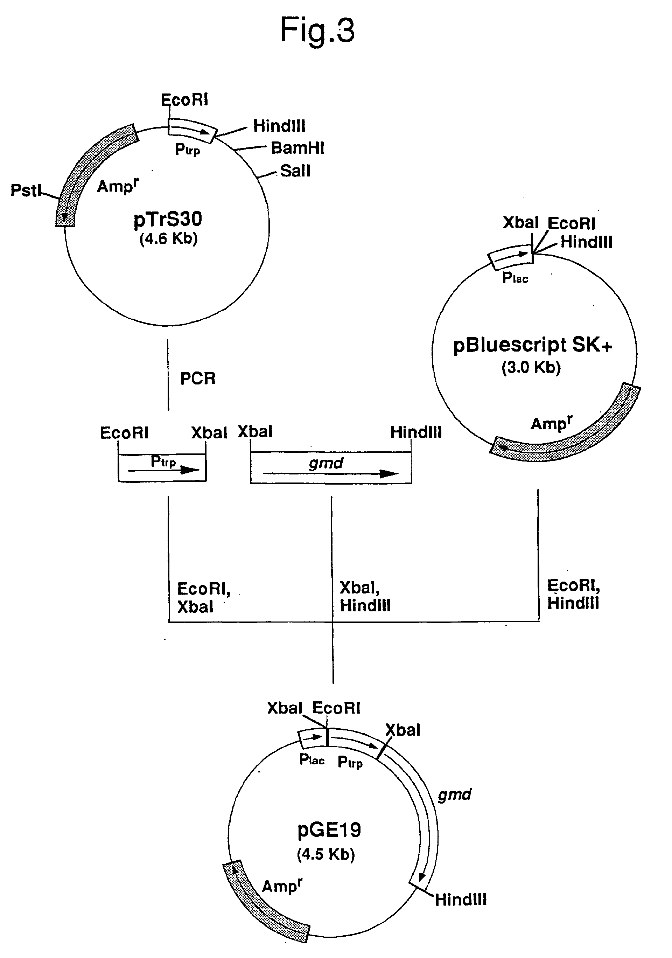

A Plasmid was extracted from the colonies of transformants grown on the medium according to the known method described above, whereby pGE19, expression plasmid, was obtained. The structure of the plasmid was confirmed by digestion with restriction enzymes (Fig. 3).

### Example 4

### Construction of a strain in which a wcaG gene derived from Escherichia coli is expressed

PCR was carried out using DNAs having nucleotide sequences shown in SEQ ID NOS: 25 and 26, respectively, which had been synthesized by using Model 8905 DNA synthesizer (PerSeptive Biosystems) as a set of primers, and chromosomal DNA of Escherichia coli W3110 (ATCC27325) as a template according to the method described in Example 1.

One-tenth of the resulting reaction mixture was subjected to agarose gel electrophoresis to confirm that the target fragment was amplified and the remaining reaction mixture was mixed with an equal amount of TE-saturated phenol/chloroform.

After centrifuging the mixture, the obtained upper layer was mixed with a two-fold volume of cold ethanol and allowed to stand at -80°C for 30 minutes. The resulting mixture was centrifuged to obtain a DNA precipitate.

The DNA precipitate was dissolved in 20 µl of TE and 5 µl of the solution was subjected to DNA cleavage with restriction enzymes ClaI and XhoI. DNA fragments were separated by agarose gel electrophoresis and 1.0 kb DNA fragment containing a wcaG gene was recovered using Gene Clean II Kit.

pPAC31 (0.2 µg) was cleaved with restriction enzymes ClaI and SalI, DNA fragments were separated by agarose gel electrophoresis and 5.2 kb fragment was recovered in the same manner as above.

The 1.0 kb, and 5.2 kb fragments were subjected to ligation reaction using a ligation kit at 16°C far 16 hours.

Escherichia coli NM522 was transformed using the ligation mixture according to the known method described above, spread on LB agar medium containing 50 µg/l ampicillin, and cultured overnight at 30°C.

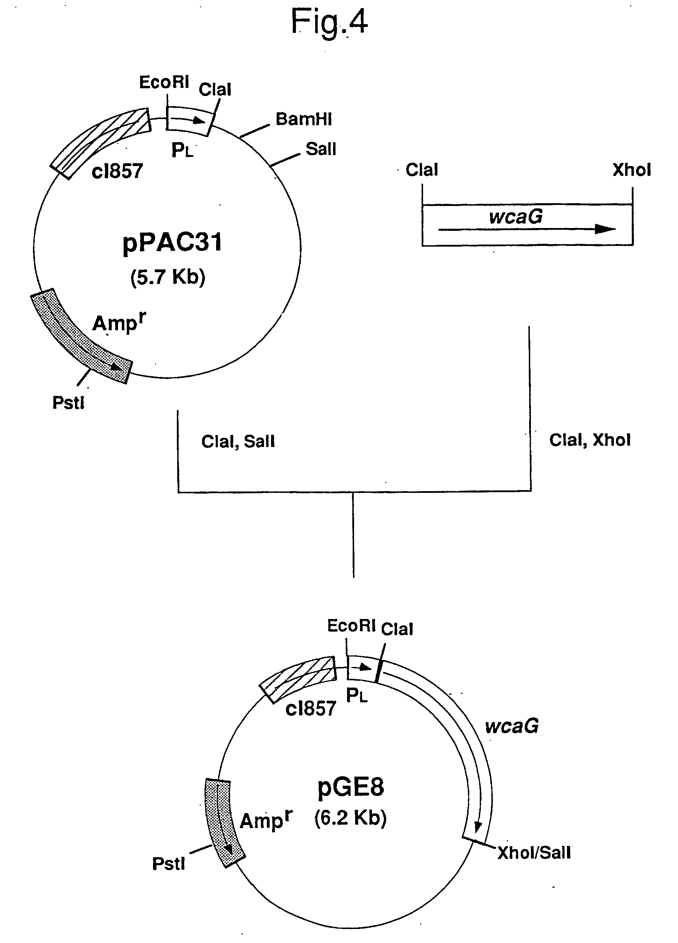

A Plasmid was extracted from the colonies of transformants grown on the medium, whereby pGE8, expression plasmid, was obtained. The structure of the plasmid was confirmed by digestion with restriction enzymes (Fig. 4).

### Example 5

### Production of 2'-fucosyllactose (1)

Escherichia coli NM522/pGT35 obtained in Example 1 was inoculated into 8 ml of LB medium containing 50 µg/ml ampicillin in a test tube and cultured at 28°C for 17 hours. The resulting culture was inoculated into 8 ml of LB medium containing 50 µg/ml ampicillin in a test tube in an amount of 1% and cultured at 37°C for 5 hours. The resulting culture (0.1 ml portion) was centrifuged to obtain wet cells. The wet cells could be stored, if necessary, at -20°C and were available for use by thawing before use.

A reaction mixture (0.1 ml) comprising the wet cells (0.1 ml portion), 50 mM citric acid buffer (pH 7.0), 10 mmol/l MnCl₂, 10 mmol/l lactose, 10 mmol/l GDP-Fuc, and 0.4% Nymeen S-215 was allowed to react at 37°C for 24 hours.

After the completion of reaction, the reaction products were analyzed using a carbohydrate analysis system (DX-500, Dionex) and it was confirmed that 0.82 mmol/l (400 mg/l) 2'-fucosyllactose was formed and accumulated in the reaction mixture.

### Example 6

### Production of 2'-fucosyllactose (2)

Escherichia coli NM522/pGT35 obtained in Example 1 was inoculated into 125 ml of LB medium containing 50 µg/ml ampicillin in a 1-L Erlenmeyer flask with baffles and cultured at 28°C for 17 hours with stirring at 220 r.p.m. The resulting culture (125 ml) was inoculated into 2.5 L of M9 medium containing 50 mg/l ampicillin in a 5-L jar fermenter and cultured at 37°C for 6 hours with stirring at 600 r.p.m. and aeration of 2.5 l/min During the culturing, the pH of the culture was maintained at 7.0 with 28% aqueous ammonia and if necessary glucose was added. The resulting culture was centrifuged to obtain wet cells. The wet cells could be stored, if necessary, at -20°C and were available for use by thawing before use.

Escherichia coli NM522/pNK11/pNT55 obtained in Example 2 was inoculated into 125 ml of LB medium [10 g/l Bacto-tryptone (Difco Laboratories Inc.), 5 g/l yeast extract (Difco Laboratories Inc.), and 5 g/l NaCl (pH 7.3)] containing 50 µg/ml ampicillin and 10 µg/ml chloramphenicol in a 1-L Erlenmeyer flask with baffles and cultured at 28°C for 17 hours with stirring at 220 r.p.m. The resulting culture (125 ml) was inoculated into 2.5 L of TB medium [10 g/l glucose, 12 g/l Bacto-tryptone (Difco Laboratories Inc.), 24 g/l yeast extract (Difco Laboratories Inc.), 2.3 g/l KH₂PO₄, and 12.5 g/l K₂HPO₄ (pH not adjusted)] containing 50 mg/l ampicillin, and 10 mg/l chloramphenicol in a 5-L jar fermenter and cultured at 30°C for 4 hours with stirring at 600 r.p.m. and aeration of 2.5 l/min, followed by culturing at 40°C for 3 hours. During the culturing, the pH of the culture was maintained at 7.0 with 28% aqueous ammonia and if necessary glucose was added. The resulting culture was centrifuged to obtain wet cells. The wet cells could be stored, if necessary, at -20°C and were available for use by thawing before use.

Escherichia coli NM522/pGE19 obtained in Example 3 was inoculated into 125 ml of LB medium containing 50 µg/ml ampicillin in a 1-L Erlenmeyer flask with baffles and cultured at 28°C for 17 hours with stirring at 220 r.p.m. The resulting culture (125 ml) was inoculated into 2.5 L of TB medium containing 50 µg/ml ampicillin in a 5-L jar fermenter and cultured at 37°C for 6 hours with stirring at 600 r.p.m. and aeration of 2.5 l/min. During the culturing, the pH of the culture was maintained at 7.0 with 28% aqueous ammonia and if necessary glucose was added. The resulting culture was centrifuged to obtain wet cells. The wet cells could be stored, if necessary, at -20°C and were available for use by thawing before use.

Escherichia coli NM522/pGE8 obtained in Example 4 was inoculated into 125 ml of LB medium containing 50 µg/ml ampicillin in a 1-L Erlenmeyer flask with baffles and cultured at 28°C for 17 hours with stirring at 220 r.p.m. The resulting culture (125 ml) was inoculated into 2.5 L of TB medium containing 50 µg/ml ampicillin in a 5-L jar fermenter and cultured at 30°C for 4 hours with stirring at 600 r.p.m. and aeration of 2.5 l/min, followed by culturing at 40°C for 3 hours. During the culturing, the pH of the culture was maintained at 7.0 with 28% aqueous ammonia and if necessary glucose was added. The resulting culture was centrifuged to obtain wet cells.

Corynebacterium ammoniagenes ATCC21170 was inoculated into 25 ml of a liquid medium comprising 50 g/l glucose , 10 g/l polypeptone (Nihon Pharmaceutical Co., Ltd.), 10 g/l yeast extract (Oriental Yeast Co., Ltd.), 5 g/l urea, 5 g/l (NH₄)₂SO₄, 1 g/l KH₂PO₄, 3 g/l K₂HPO₄, 1 g/l MgSO₄·7H₂O, 0.1 g/l CaCl₂·2H₂O, 10 mg/l FeSO₄·7H₂O, 10 mg/l ZnSO₄·7H₂O, 20 mg/l MnSO₄·4-6H₂O, 20 mg/l L-cystein, 10 mg/l D-calcium pantothenate, 5 mg/l vitamin B1, 5 mg/l nicotinic acid and 30 µg/l biotin (adjusted to pH 7.2 with 10 mol/l NaOH) in a 300 ml-Erlenmeyer flask with baffles and cultured at 28°C with stirring at 220 r.p.m. for 24 hours.

The resulting culture (20 ml) was inoculated into 250 ml of a liquid medium having the same composition as above in a 2-L Erlenmeyer flask with baffles and cultured at 28°C with stirring at 220 r.p.m. for 24 hours. The obtained culture was used as a seed culture.

The seed culture (250 ml) was inoculated into 2.25 L of a liquid medium comprising 150 g/l glucose, 5 g/l meat extract (Kyokuto Seiyaku), 10 g/l KH₂PO₄, 10 g/l K₂HPO₄, 10 g/l MgSO₄·7H₂O, 0.1 g/l CaCl₂·2H₂O, 20 mg/l FeSO₄·7H₂O, 10 mg/l ZnSO₄·7H₂O, 20 mg/l MnSO₄·4-6H₂O (separately sterilized), 15 mg/l β-alanine (separately sterilized), 20 mg/l L-cystein, 100 µg/l biotin, 2 g/l urea, and 5 mg/l vitamin B1 (separately sterilized) (adjusted to pH 7.2 with 10 mol/l NaOH) in a 5-L jar fermenter and cultured at 32°C with stirring at 600 r.p.m. and aeration of 2.5 l/min for 24 hours. During the culturing, the pH of the culture was maintained at 6.8 with 28% aqueous ammonia.

The resulting culture was centrifuged to obtain wet cells. The wet cells could be stored, if necessary, at -20°C and were available for use by thawing before use.

A reaction mixture (30 ml) comprising 25 g/l Escherichia coli NM522/pNK11/pNT55 wet cells, 15 g/l Escherichia coli NM522/pGE19 wet cells, 15 g/l Escherichia coli NM522/pGE8 wet cells, 50 g/l Escherichia coli NM522/pGT35 wet cells, 150 g/l Corynebacterium ammoniagenes ATCC21170 wet cells, 100 g/l fructose, 30 g/l mannose, 100 g/l lactose, 30 g/l GMP, 25/1 KH₂PO₄, 5 g/l MgSO₄·7H₂O, 5 g/l phytic acid, 4 g/l Nymeen S-215, and 10 ml/l xylene was put into a 200-ml beaker and allowed to react at 32°C for 48 hours while stirring with a magnetic stirrer (900 r.p.m.). During the culturing, the pH of the reaction mixture was maintained at 7.2 with 4 mol/l NaOH and if necessary, fructose and KH₂PO₄ were added.

After the completion of reaction, the reaction products were analyzed using HPLC and it was confirmed that 13.4 g/l 2'-fucosyllactose was formed and accumulated in the reaction mixture.

### Example 7

### Production of lacto-N-neodifucohexaose I

Escherichia coli NM522/pGT35 obtained in Example 1 was inoculated into 8 ml of LB medium containing 50 µg/ml ampicillin in a test tube and cultured at 28°C for 17 hours.

The resulting culture was inoculated into 8 ml of LB medium containing 50 µg/ml ampicillin in a test tube in an amount of 1% and cultured at 37°C for 5 hours.

After the completion of culturing, 0.1 ml portion of the culture was centrifuged to obtain wet cells. The wet cells could be stored, if necessary, at -20°C and were available for use by thawing before use.

A reaction mixture (0.1 ml) comprising the wet cells (0.1 ml portion), 50 mmol/l citric acid buffer (pH 7.0), 10 mmol/l MnCl₂, 10 mmol/l GDP-Fucose, 0.4% Nymeen S-215 and 10 m mol/l lacto-N-neofucopentaoseIII (LNFPIII) was allowed to react at 37°C for 24 hours.

After the completion of reaction, the reaction products were analyzed using a carbohydrate analysis system (DX-500, Dionex) and it was confirmed that 2.9 mmol/l (1.4 g/l) lacto-N-neodifucohexaose I was formed and accumulated in the reaction mixture.

### Industrial Applicability

According to the present invention, α1,2-fucosyltransferase can be produced in large quantities by recombinant DNA techniques. Fucose-containing carbohydrates such as 2'-fucosyllactose can be efficiently produced by using the enzyme.

### [Sequence Listing Free Text]

- SEQ ID NO: 1 -: Description of the artificial sequence: synthetic DNA
- SEQ ID NO: 3 -: Description of the artificial sequence: synthetic DNA
- SEQ ID NO: 4 -: Description of the artificial sequence: synthetic DNA
- SEQ ID NO: 5 -: Description of the artificial sequence: synthetic DNA
- SEQ ID NO: 6 -: Description of the artificial sequence: synthetic DNA
- SEQ ID NO: 7 -: Description of the artificial sequence: synthetic DNA
- SEQ ID NO: 8 -: Description of the artificial sequence: synthetic DNA
- SEQ ID NO: 9 -: Description of the artificial sequence: synthetic DNA
- SEQ ID NO: 10 -: Description of the artificial sequence: synthetic DNA
- SEQ ID NO: 11 -: Description of the artificial sequence: synthetic DNA
- SEQ ID NO: 12 -: Description of the artificial sequence: synthetic DNA
- SEQ ID NO: 13 -: Description of the artificial sequence: synthetic DNA
- SEQ ID NO: 14 -: Description of the artificial sequence: synthetic DNA
- SEQ ID NO: 15 -: Description of the artificial sequence: synthetic DNA
- SEQ ID NO: 16 -: Description of the artificial sequence: synthetic DNA
- SEQ ID NO: 17 -: Description of the artificial sequence: synthetic DNA
- SEQ ID NO: 18 -: Description of the artificial sequence: synthetic DNA
- SEQ ID NO: 19 -: Description of the artificial sequence: synthetic DNA
- SEQ ID NO: 20 -: Description of the artificial sequence: synthetic DNA
- SEQ ID NO: 21 -: Description of the artificial sequence: synthetic DNA
- SEQ ID NO: 22 -: Description of the artificial sequence: synthetic DNA
- SEQ ID NO: 23 -: Description of the artificial sequence: synthetic DNA
- SEQ ID NO: 24 -: Description of the artificial sequence: synthetic DNA
- SEQ ID NO: 25 -: Description of the artificial sequence: synthetic DNA
- SEQ ID NO: 26 -: Description of the artificial sequence: synthetic DNA

## Claims

1. A DNA encoding a protein having α1,2-fucosyltransferase activity,
said DNA having a nucleotide sequence wherein one or more nucleotide residues in one or more members selected from the group consisting of (a) sequences, (b) sequences and (c) sequences have been deleted, substituted or added:
(a) poly-C sequences,
(b) TAA-analogous repeat sequences,
(c) AAAAAAG sequences, and
(a), (b) and (c) sequences being partial in a DNA encoding a protein having α1,2-fucosyltransferase activity derived from Helicobacter pylori.

2. The DNA according to claim 1, wherein the substitution is a substitution in which one or more codons are modified into codons which are used with a high frequency in a host cell in which the DNA is expressed.

3. A DNA encoding a protein having α1,2-fucosyltransferase activity,
wherein said DNA is obtained by modifying one or more codons which are partial in a DNA encoding a protein having α1,2-fucosyltransferase activity derived from Helicobacter pylori into codons which are used with a high frequency in a host cell in which said DNA is expressed.

4. The DNA according to claim 3, wherein the modification is a modification in one or more members selected from the group consisting of (a) sequences, (b) sequences and (c) sequences selected from:
(a) poly-C sequences,
(b) TAA-analogous repeat sequences,
(c) AAAAAAG sequences, and
(a), (b) and (c) sequences being partial in a DNA encoding a protein having α1,2-fucosyltransferase activity derived from Helicobacter pylori.

5. The DNA according to claim 3, wherein the DNA comprising a nucleotide sequence which is obtained by modifying all codons into codons which are used with a high frequency in a host cell in which said DNA is expressed.

6. A DNA comprising the nucleotide sequence represented by SEQ ID NO: 1.

7. A DNA comprising a nucleotide sequence wherein one or more nucleotide residues have been deleted, substituted or added in the DNA represented by the nucleotide sequence shown in SEQ ID NO: 1 and encoding a protein having α1,2-fucosyltransferase activity.

8. A recombinant DNA which is obtained by ligating DNA according to any one claims 1 to 7 with a vector.

9. A transformant which is obtained by introducing the recombinant DNA according to claim 8 into a host cell.

10. The transformant according to claim 9, wherein the host cell is a microorganism.

11. The transformant according to claim 10, wherein the microorganism is a microorganism belonging to the genus Escherichia.

12. The transformant according to claim 11, wherein the microorganism belonging to the genus Escherichia is Escherichia coli.

13. A process for producing a protein having α1,2-fucosyltransferase activity which comprises culturing a transformant according to any one of claims 9 to 12 in a medium, allowing the protein having α1,2-fucosyltransferase activity to be formed and accumulated in the culture and recovering the protein from the culture.

14. A process for producing a fucose-containing carbohydrate which comprises allowing a culture of a transformant according to any one of claims 9 to 12 or a treated matter thereof as an enzyme source, an acceptor carbohydrate, and guanosine diphosphofucose (hereinafter referred to as GDP-fucose) to be present in an aqueous medium; transferring fucose to the acceptor carbohydrate by α1,2 linkage to form and accumulate the fucose-containing carbohydrate in the aqueous medium; and recovering the fucose-containing carbohydrate from the aqueous medium.

15. The process for producing a fucose-containing carbohydrate according to claim 14, which comprises allowing the following enzyme sources;
(a) a culture of a microorganism capable of forming guanosine-5'-triphosphate (hereinafter referred to as GTP) from a precursor of GTP or a treated matter thereof,
(b) a culture of a microorganism capable of forming GDP-fucose from a sugar and GTP or a treated matter thereof, and
(c) a culture of a transformant selected from the transformant according to any one of claims 9 to_12 or a treated matter thereof,
the precursor, a sugar, and an acceptor carbohydrate to be present in an aqueous medium;
allowing the fucose-containing carbohydrate to be formed and accumulated in the aqueous medium; and
recovering the fucose-containing carbohydrate from the aqueous medium.

16. The process for producing a fucose-containing carbohydrate according to claim 14 or 15, wherein the acceptor carbohydrate is a carbohydrate containing an oligosaccharide consisting of 10 or less monosaccharides having galactose at its non-reducing terminal.

17. The process for producing a fucose-containing carbohydrate according to claim 16, wherein the oligosaccharide is lactose, N-acetyllactosamine, Lewis X or Lewis a.

18. The process for producing a fucose-containing carbohydrate according to claim 14 or 15, wherein the fucose-containing carbohydrate is fucosyllactose, fucosyl-N-acetyllactosamine, Lewis Y or Lewis b.

19. The process for producing a fucose-containing carbohydrate according to claim 14 or 15, wherein the treated matter of the culture is a concentrated culture, dried culture, a cell obtained by centrifuging the culture, a cell obtained by drying or freeze drying the cell, a product obtained by treating the cell with a surfactant or by ultrasonication or mechanical friction of the cell or by treating the cell with a solvent or an enzyme or by protein-fractionation or immobilization of the cell, or an enzyme preparation obtained by extraction from the cell.

20. The process for producing a fucose-containing carbohydrate according to claim 15, wherein the precursor is guanine, xanthine, hypoxanthine, guanosine, xanthosine, inosine, guanosine-5'-monophosphate, xanthosine-5'-monophosphate or inosine-5'-monophosphate.

21. The process for producing a fucose-containing carbohydrate according to claim 15, wherein the sugar is a sugar selected from the group consisting of glucose, fructose and mannose.

22. The process for producing a fucose-containing carbohydrate according to claim 15, wherein the microorganism capable of forming GTP from a precursor is a microorganism selected from microorganisms belonging to the genus Corynebacterium.

23. The process for producing a fucose-containing carbohydrate according to claim 22, wherein the microorganisms belonging to the genus Corynebacterium are Corynebacterium ammoniagenes.

24. The process for producing a fucose-containing carbohydrate according to claim 15, wherein forming GDP-fucose from a sugar and GTP is carried out by one or more microorganisms.

25. The process for producing a fucose-containing carbohydrate according to claim 24, wherein the microorganisms are selected from microorganisms belonging to the genus Echerichia or Corynebacterium.

26. The process for producing a fucose-containing carbohydrate according to claim 25, wherein the microorganisms belonging to the genus Escherichia are Escherichia coli.

27. The process for producing a fucose-containing carbohydrate according to claim 25, wherein the microorganisms belonging to the genus Corynebacterium are Corynebacterium ammoniagenes.

28. The process for producing a fucose-containing carbohydrate according to claim 15, wherein the microorganism capable of forming GDP-fucose from a sugar and GTP is a microorganism having a strong activity of one or more enzymes selected from the group consisting of glucokinase, phosphomannomutase, mannose-1-phosphoguanylyltransferase, phosphoglucomutase, phosphofructokinase, GDP-mannose 4,6-dehydratase, and GKDM epimerase/reductase.

29. The process for producing a fucose-containing carbohydrate according to claim 28, wherein the microorganism comprises one or more microorganisms containing recombinant DNA which comprises a DNA fragment containing one or more genes selected from the group consisting of a gene encoding glucokinase (hereinafter referred to as glk gene), a gene encoding phosphomannomutase (hereinafter referred to as manB gene), a gene encoding mannose-1-phosphoguanyltransferase (hereinafter referred to as manC gene), a gene encoding phosphoglucomutase (hereinafter referred to as pgm gene), a gene encoding phosphofructokinase (hereinafter referred. to as pfk gene), a gene encoding GDP-mannose 4,6-dehydratase (hereinafter referred to as gmd gene), and a gene encoding GKDM epimerase/reductase (hereinafter referred to as wcaG gene) and a vector.

30. The process for producing a fucose-containing carbohydrate according to claim 29, wherein the glk gene, the manB gene, the manC gene, the pgm gene, the pfk gene, the gmd gene and the wcaG gene are genes derived from Escherichia coli.
